(19) Europäisches Patentamt
European Patent Office
Office européen des brevets

(11) EP 2 189 807 A2

(12) **EUROPEAN PATENT APPLICATION**

(43) Date of publication:
26.05.2010 Bulletin 2010/21

(51) Int Cl.:
*G01S 7/52* (2006.01)

(21) Application number: 09174271.8

(22) Date of filing: 28.10.2009

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **19.11.2008 KR 20080115316**

(71) Applicant: **Medison Co., Ltd.**
**Kangwon-do 250-870 (KR)**

(72) Inventors:
• **Lee, Jin Yong**
**135-851 Seoul (KR)**
• **Kim, Jae Gyoung**
**135-851 Seoul (KR)**

(74) Representative: **Schmid, Wolfgang**
**Lorenz & Kollegen**
**Patentanwälte Partnerschaftsgesellschaft**
**Alte Ulmer Straße 2**
**89522 Heidenheim (DE)**

(54) **Finding a standard view corresponding to an acquired ultrasound image**

(57) Embodiments for automatically finding a standard view corresponding to an acquired ultrasound image of a target object in an ultrasound system (100) are disclosed. A mapping table associating information on a plurality of standard views of a target object with predetermined first feature vectors is stored in the storage unit (110). The predetermined first feature vectors may be previously calculated from the typical plane images corresponding to the standard views. A processing unit (140) calculates a second feature vector form an acquired ultrasound image, and refers to the mapping table to select one of the first feature vectors closest to the second feature vector. The processing unit (140) extracts information on the standard view corresponding to the selected first feature vector from the mapping table.

FIG. 1

**Description**

The present application claims priority from Korean Patent Application No. 10-2008-0115316 filed on November 19, 2008, the entire subject matter of which is incorporated herein by reference.

## TECHNICAL FIELD

The present disclosure generally relates to ultrasound systems, and more particularly to an ultrasound system and method of automatically finding a standard view corresponding to an acquired ultrasound image.

## BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional diagnostic images of internal features of an object (e.g., human organs).

The plane images provided by the ultrasound system may be generally classified into various types of standard views, such as a parasternal view, an apical view, a subcostal view, a suprasternal view, etc. may be acquired in the ultrasound system. Conventionally, the view type of the heart is manually selected by the user. Thus, the user may commit an error in selecting a standard view for desirable observation, so that the ultrasound image may not be adequately observed.

## SUMMARY

Embodiments for testing an acoustic property of an ultrasound probe including a plurality of transducer elements are disclosed herein. In one embodiment, by way of non-limiting example, an ultrasound system comprises: a storage unit to store a mapping table associating information on a plurality of standard views of a target object with predetermined first feature vectors; an ultrasound data acquisition unit operable to transmit ultrasound signals to the target object and receive echo signals reflected from the target object, the ultrasound data acquisition unit being further operable to from ultrasound data based on the receive echo signals; an ultrasound image forming unit operable to form an ultrasound image based on the ultrasound data; and a processing unit operable to calculate a second feature vector from the ultrasound image and refer to the mapping table to select one of the predetermined first feature vectors closest to the second feature vector, the processing unit being further operable to extract information on the standard view corresponding to the selected predetermined first feature vector from the mapping table.

In another embodiment, a method of providing information on one of a plurality of standard views of a target object in an ultrasound system, comprises: a) storing a mapping table associating information on a plurality of standard views of a target object with predetermined first feature vectors; b) transmitting ultrasound signals to the target object and receive echo signals reflected from the target object to from ultrasound data based on the receive echo signals; c) forming an ultrasound image based on the ultrasound data; and d) calculating a second feature vector from the ultrasound image; e) referring to the mapping table to select one of the first feature vectors closest to the second feature vector to extract information on the standard view corresponding to the selected predetermined first feature vector from the mapping table.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

## BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a flowchart showing a procedure of forming a mapping table associating information on standard views of a target object with predetermined feature vectors.
FIG. 4 is a schematic diagram showing examples of typical plane images corresponding to standard views.
FIG. 5 is a block diagram showing an illustrative embodiment of a processing unit.
FIG. 6 is a schematic diagram showing an example of an ultrasound image.

## DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

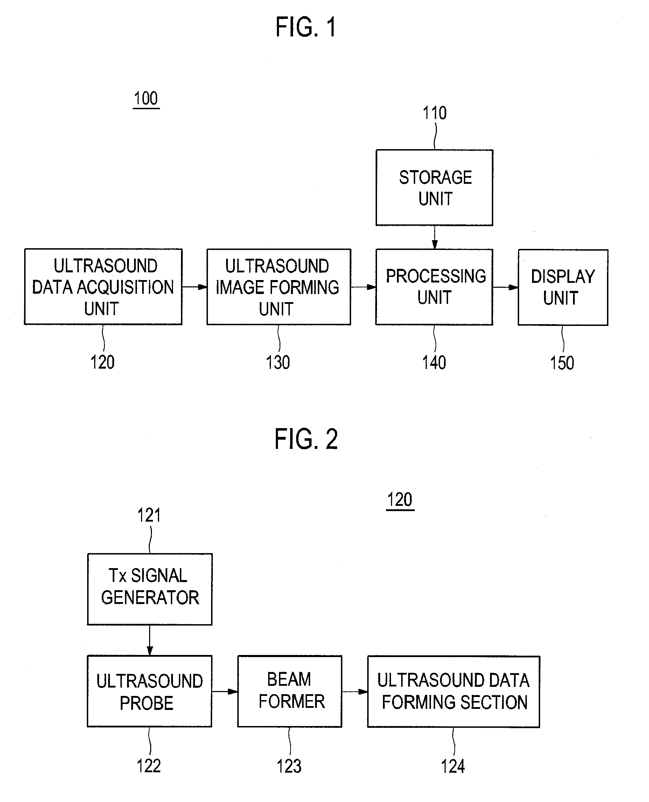

FIG. 1 is a block diagram showing an illustrative embodiment of an ultrasound system. As depicted therein, the ultrasound system 100 may include a storage unit 110 that may store a mapping table associating information upon a plurality of standard views of a target object with first feature vectors, which are previously calculated from typical plane images corresponding to the respective standard views. The detailed description of calculating the first feature vectors from the plane images will be described later.

In one embodiment, the target object may include a heart, and the standard views may include a parasternal view, an apical view, a subcostal view, a suprasternal view, etc.

The ultrasound system 100 may further include an ultrasound data acquisition unit 120. The ultrasound data acquisition unit 120 may be operable to transmit/receive ultrasound signals to/from the target object to thereby form ultrasound data.

Referring to FIG. 2, the ultrasound data acquisition unit 120 may include a transmit (Tx) signal generator 121 that may be operable to generate a plurality of Tx signals. The ultrasound data acquisition unit 120 may further include an ultrasound probe 122 coupled to the Tx signal generator 121. The ultrasound probe 122 may be operable to transmit the ultrasound signals to the target object in response to the Tx signals. The ultrasound probe 122 may be further operable to receive echo signals reflected from the target object to thereby form electrical receive signals. The ultrasound probe 122 may contain an array transducer consisting of a plurality of transducer elements. The array transducer may include a 1D or 2D array transducer, but is not limited thereto. The array transducer may be operable to generate ultrasound signals and convert the echo signals into the electrical receive signals.

The ultrasound data acquisition unit 120 may further include a beam former 123. The beam former 123 may be operable to apply delays to the electrical receive signals in consideration of positions of the transducer elements and focal points. The beam former 123 may further be operable to sum the delayed receive signals to thereby output a plurality of receive-focused beams. The ultrasound data acquisition unit 120 may further include an ultrasound data forming section 124 that may be operable to form the ultrasound data based on the receive-focused beams. In one embodiment, the ultrasound data may be radio frequency data or in-phase/quadrature data.

Referring back to FIG. 1, the ultrasound system 100 may further include an ultrasound image forming unit 130. The ultrasound image forming unit 130 may be operable to form an ultrasound image based on the ultrasound data formed in the ultrasound data acquisition unit 110. In one embodiment, by way of non-limiting example, the ultrasound image may be a brightness mode image. The ultrasound image may be also stored in the storage unit 110.

The ultrasound system 100 may further include a processing unit 140. The processing unit 140 may be operable to calculate a second feature vector from the ultrasound image formed in the ultrasound image forming unit 130. The processing unit 140 may be further operable to compute an Euclidean distance between the second feature vector and each of the first feature vectors of the mapping table stored in the storage unit 110. The processing unit 140 may be further operable to select one of the first feature vectors, which has the closest Euclidean distance, and access the storage unit 110 to find one of the standard views corresponding to the selected first feature vector from the mapping table. The found information may be displayed on a screen of a display unit 150.

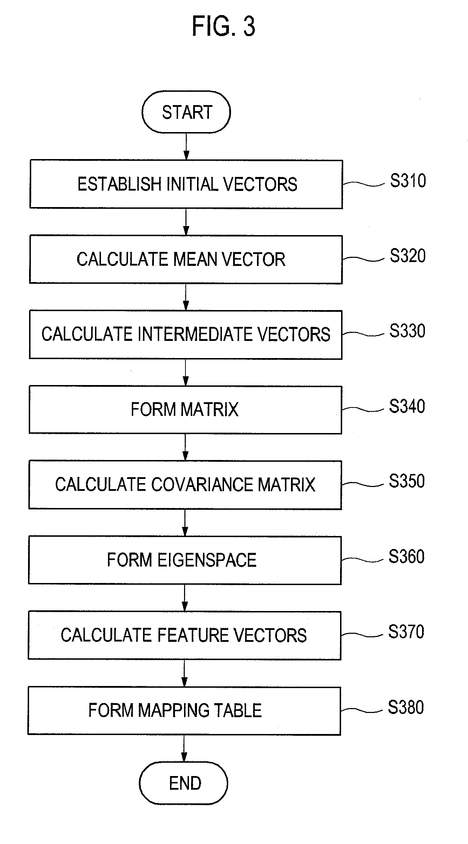

Hereinafter, the detained description of calculating the first feature vectors from the typical plane images corresponding to the respective standard views will follow with reference to FIG. 3. In one embodiment, by way of non-limiting example, the feature vectors may be calculated by using statistical algorithm, such as principle component analysis, etc.

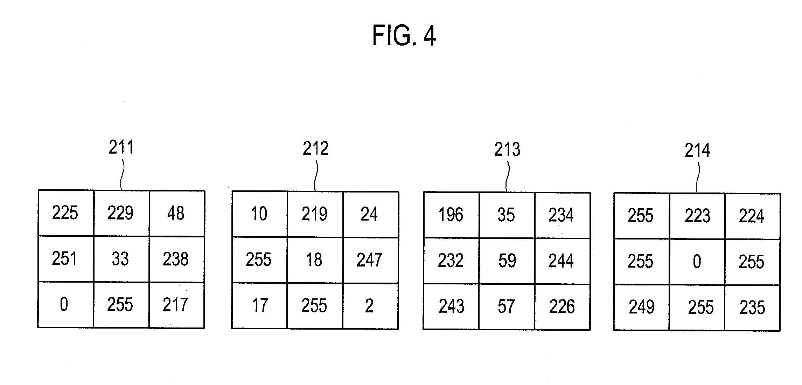

FIG. 3 is a flowchart showing a procedure of forming a mapping table associating information on the standard views of a target object with the first feature vectors calculated from the typical plane images corresponding to the standard views. As illustrated in FIG. 3, initial vectors may be established from the plurality of plane images corresponding to the respective standard views of the target object at S310. In one embodiment, the initial vectors may be established by transforming pixel values of each plane image into a 1-dimensional matrix (i.e., 1 N), wherein N is the number of the pixels. For example, assuming that four plane images 211-214 corresponding to the parasternal view, apical view, subcostal view and suprasternal view are provided, as shown in FIG. 4, four initial vectors $x_1$-$x_4$ corresponding to the respective plane images 211-214 may be established by sequentially taking the pixel values in each of the plane images 211-214 in a horizontal direction to form a column vector, as follows:

$$x_1 = \begin{bmatrix} 225 \\ 229 \\ 48 \\ 251 \\ 33 \\ 238 \\ 0 \\ 255 \\ 217 \end{bmatrix} \quad x_2 = \begin{bmatrix} 10 \\ 219 \\ 24 \\ 255 \\ 18 \\ 247 \\ 17 \\ 255 \\ 2 \end{bmatrix} \quad x_3 = \begin{bmatrix} 196 \\ 35 \\ 234 \\ 232 \\ 59 \\ 244 \\ 243 \\ 57 \\ 226 \end{bmatrix} \quad x_4 = \begin{bmatrix} 255 \\ 223 \\ 224 \\ 255 \\ 0 \\ 255 \\ 249 \\ 255 \\ 235 \end{bmatrix} \tag{1}$$

Thereafter, a mean vector of the initial vectors $x_1$-$x_4$ may be calculated at S320, and then the mean vector may be stored in the storage unit 110. The mean vector may be calculated through the following equation.

$$m = \frac{1}{P} \sum_{i=1}^{P} x_i \tag{2}$$

where m represent the mean vector, and P represents the number of the initial vectors x1-x4. For example, when the equation (2) is applied to the initial vectors $x_1$-$x_4$, the following mean vector may be obtained.

$$m = \begin{bmatrix} 171.50 \\ 176.50 \\ 135.5 \\ 248.25 \\ 27.50 \\ 246.00 \\ 127.25 \\ 205.50 \\ 170.00 \end{bmatrix} \tag{3}$$

Subsequently, the mean vector m may be subtracted from each of the initial vectors $x_1$-$x_4$ to thereby obtain intermediate vectors $\overline{x_1}$ - $\overline{x_4}$. The intermediate vectors $\overline{x_1}$ - $\overline{x_4}$ may be represented as follows:

$$\overline{x}_1 = \begin{bmatrix} 53.50 \\ 52.50 \\ -84.50 \\ 2.75 \\ 5.50 \\ -8.00 \\ -127.25 \\ 49.50 \\ 47.00 \end{bmatrix} \quad \overline{x}_2 = \begin{bmatrix} -161.50 \\ 42.50 \\ -108.50 \\ 6.75 \\ -9.50 \\ 1.00 \\ -110.25 \\ 49.50 \\ -168.00 \end{bmatrix} \quad \overline{x}_3 = \begin{bmatrix} 24.50 \\ -141.50 \\ 101.50 \\ -16.25 \\ 31.50 \\ -2.00 \\ 115.75 \\ -148.50 \\ 56.00 \end{bmatrix} \quad \overline{x}_4 = \begin{bmatrix} 83.50 \\ 46.50 \\ 91.50 \\ 6.75 \\ -27.50 \\ 9.00 \\ 121.75 \\ 49.50 \\ 65.00 \end{bmatrix} \quad (4)$$

An intermediate matrix $\overline{x}$ may be obtained by using the intermediate vectors $\overline{x}_1$ - $\overline{x}_4$ at S340. The intermediate matrix $\overline{x}$ may be expressed as follows:

$$\overline{X} = \begin{bmatrix} 53.50 & -161.50 & 24.50 & 83.50 \\ 52.50 & 42.50 & -141.50 & 46.50 \\ -84.50 & -108.50 & 101.50 & 91.50 \\ 2.75 & 6.75 & -16.25 & 6.75 \\ 5.50 & -9.50 & 31.50 & -27.50 \\ -8.00 & 1.00 & -2.00 & 9.00 \\ -127.25 & -110.25 & 115.75 & 121.75 \\ 49.50 & 49.50 & -148.50 & 49.50 \\ 47.00 & -168.00 & 56.00 & 65.00 \end{bmatrix} \quad (5)$$

A covariance matrix $\Omega$ may be calculated from the intermediate matrix $\overline{x}$ at S350. The covariance matrix $\Omega$ may be calculated as follows:

$$\Omega = \overline{X}\,\overline{X}^T = \begin{bmatrix} 36517 & -3639 & 23129 & -778 & 304 & 113 & 24000 & -4851 & 36446 \\ -3639 & 26747 & -19155 & 3045 & -5851 & 324 & -22083 & 28017 & -9574 \\ 23129 & -19155 & 37587 & -1997 & 1247 & 1188 & 45603 & -20097 & 25888 \\ -778 & 3045 & -1996 & 363 & -746.5 & 78 & -2153 & 3217 & -1476 \\ 304 & -5851 & 1247 & -747 & 1869 & -364 & 645 & -6237 & 1831 \\ 113 & 324 & 1188 & 78 & -364 & 150 & 1772 & 396 & -71 \\ 24000 & -22083 & 45603 & -2153 & 645.5 & 1772 & 56569 & -22919 & 26937 \\ -4851 & 28017 & -20097 & 3218 & -6237 & 396 & -22919 & 29403 & -11088 \\ 36446 & -9574 & 25888 & -1476 & 1831 & -71 & 26937 & -11088 & 37794 \end{bmatrix} \quad (6)$$

Subsequently, eigenvalues may be calculated from the covariance matrix $\Omega$, and then eigenvectors corresponding to the respective eigenvalues may be calculated. In one embodiment, by way of non-limiting example, the eigenvalues and the eigenvectors may be calculated by using Jacobi algorithm. The eigenvectors may be structured to a matrix, which may represent an eigenspace, at S380. For example, the eigenvalues λ1- λ3 and the eigenvectors $v_1$-$v_3$ of the covariance matrix Ω may be calculated as follows:

$$\lambda1 = 153520 \qquad \lambda2 = 50696 \qquad \lambda3 = 22781$$

$$v_1 = \begin{bmatrix} 0.356 \\ -0.279 \\ 0.480 \\ -0.031 \\ 0.035 \\ 0.009 \\ 0.560 \\ -0.296 \\ 0.402 \end{bmatrix} \qquad v_2 = \begin{bmatrix} -0.552 \\ -0.489 \\ 0.044 \\ -0.048 \\ 0.105 \\ -0.004 \\ 0.112 \\ 0.492 \\ -0.432 \end{bmatrix} \qquad v_3 = \begin{bmatrix} -0.264 \\ 0.347 \\ 0.309 \\ 0.064 \\ -0.222 \\ 0.078 \\ 0.585 \\ 0.401 \\ -0.391 \end{bmatrix} \tag{7}$$

The eigenspace V may be formed by using the eigenvectors $v_1$-$v_3$, as follows:

$$V = \begin{bmatrix} 0.356 & -0.552 & -0.264 \\ -0.279 & -0.489 & 0.347 \\ 0.480 & 0.044 & 0.309 \\ -0.031 & -0.048 & 0.064 \\ 0.035 & 0.105 & -0.222 \\ 0.009 & -0.004 & 0.078 \\ 0.560 & 0.112 & 0.585 \\ -0.296 & 0.492 & 0.401 \\ 0.402 & -0.432 & -0.391 \end{bmatrix} \tag{8}$$

Thereafter, at S370, the first feature vectors of the respective plane images corresponding to the respective standard views may be calculated by using the intermediate vectors calculated at S330 and the eigenspace formed at S360. In one embodiment, by way of non-limiting example, the intermediate vectors $\overline{x_1}$ - $\overline{x_4}$ may be projected into the eigenspace V to thereby obtain the feature vectors $\hat{x}_1$ - $\hat{x}_4$, as follows:

$$\hat{x}_1 = V^T \overline{x_1} = \begin{bmatrix} -103.09 \\ -117.31 \\ -96.57 \end{bmatrix} \qquad \hat{x}_2 = V^T \overline{x_2} = \begin{bmatrix} -265.92 \\ 98.29 \\ 47.45 \end{bmatrix}$$

$$\hat{x}_3 = V^T \overline{x_3} = \begin{bmatrix} 229.76 \\ 125.90 \\ -46.14 \end{bmatrix} \qquad \hat{x}_4 = V^T \overline{x_4} = \begin{bmatrix} 139.24 \\ -106.88 \\ 95.26 \end{bmatrix} \tag{9}$$

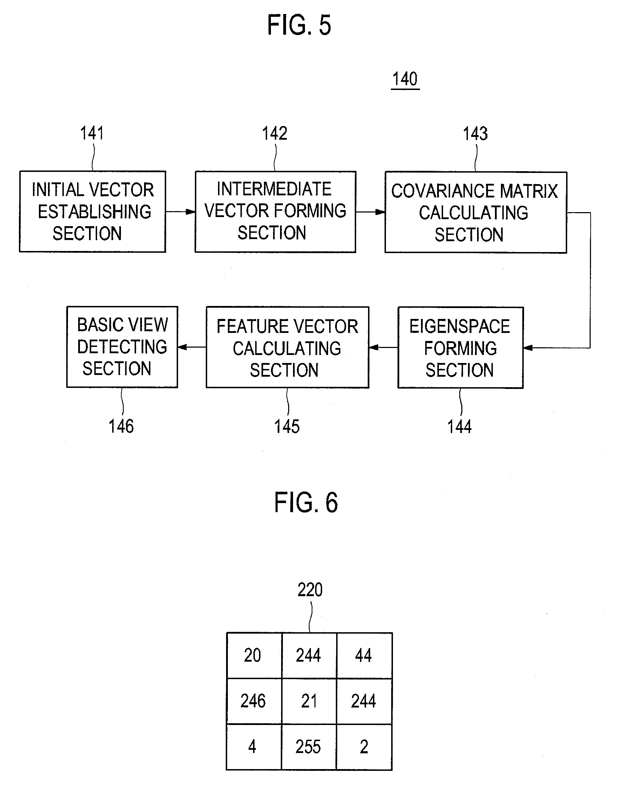

FIG. 5 is a block diagram showing an illustrative embodiment of the processing unit 140. The processing unit 140 may include an initial vector establishing section 141. The initial vector establishing section 141 may be operable to establish an initial vector based on the pixel values of the ultrasound image provided from the ultrasound image forming unit 130. In one embodiment, the initial vector establishing section 141 may be operable to establish the initial vector by transforming pixel values of the ultrasound image into a 1-dimensional matrix (i.e., 1 N), wherein N is the number of the pixels. For example, assuming that an ultrasound image 600 is provided, as shown in FIG. 6, the initial vector y may be established by taking the pixel values in the ultrasound image 600 in a horizontal direction, as follows:

$$y = \begin{bmatrix} 20 \\ 244 \\ 44 \\ 246 \\ 21 \\ 244 \\ 4 \\ 255 \\ 2 \end{bmatrix} \qquad (10)$$

The processing unit 140 may further include an intermediate vector calculating section 142. The intermediate vector forming section 142 may be operable to subtract a mean vector from the initial vector to thereby obtain an intermediate vector. In one embodiment, the stored mean vector in the storage unit 110 may be used as the mean vector. For example, the intermediate vector calculating section 142 may be operable to obtain the intermediate vector $\bar{y}$, as follows:

$$\bar{y} = \begin{bmatrix} -151.50 \\ 67.50 \\ -88.5 \\ -2.25 \\ -6.50 \\ -2.00 \\ -123.25 \\ 49.50 \\ -168.00 \end{bmatrix} \qquad (11)$$

The processing unit 140 may further include a covariance matrix calculating section 143. The covariance matrix calculating section 143 may be operable to calculate a covariance matrix from the intermediate matrix. The calculation of the covariance matrix may be performed in the same manner with the operation of S350 in FIG. 3. Thus, the detailed description of calculating the covariance matrix may be omitted herein.

The processing unit 140 may further include an eigenspace forming section 144. The eigenspace forming section 144 may be operable to calculate eigenvalues and eigenvectors from the covariance matrix calculated in the covariance matrix calculating section 143. In one embodiment, by way of non-limiting example, the eigenvalues and the eigenvectors may be calculated by using Jacobi algorithm. The eigenspace forming section 144 may be further operable to form an eigenspace by using the eigenvalues and eigenvectors. The eigenspace forming section 144 may be operable to form the eigenspace similar to the operation of S360 in FIG. 3.

The processing unit 140 may further include a feature vector calculating section 145. The feature vector calculating section 145 may be operable to calculate a second feature vector by using the intermediate vector calculated in the intermediate vector forming section 142 and the eigenspace formed in the Eigen space forming section 144. In one embodiment, by way of non-limiting example, the feature vector calculating section 145 may be operable to project the intermediate vector $\bar{x}$ may into the Eigen space V to thereby obtain the second feature vector $\hat{y}$, as follows:

$$\hat{y} = V^T \bar{y} = \begin{bmatrix} -266.65 \\ 80.75 \\ 50.60 \end{bmatrix} \qquad (12)$$

The processing unit 140 may further include a standard view detecting section 146. The standard view detecting section 146 may be operable to retrieve the mapping table to detect a standard view corresponding to the second feature vector calculated in the feature vector calculating section 145. In one embodiment, the standard view detecting section 146 may be operable to compute an Euclidean distance between the second feature vector and each of the first feature vectors of the mapping table stored in the storage unit 110 to detect the corresponding standard view. That is, the standard view detecting section 146 may be operable to select one of the first feature vectors, which has the closest Euclidean distance, and access the storage unit 100 to find one of the standard views corresponding to the selected first feature vector from the mapping table. The extracted information may be displayed on a screen of the display unit 150.

Although the ultrasound image forming unit 130 and the processing unit 140 are described in different elements in one embodiment, the ultrasound image forming unit 130 and the processing unit 140 may be embodied in a single processor. Also, although it is described above that the feature vectors are calculated by using principle component analysis in one embodiment, the feature vectors may be calculated by other statistical algorithm, such as Hidden Markove model, support vector machine algorithm, etc.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1.    An ultrasound system, comprising:

   a storage unit to store a mapping table associating information on a plurality of standard views of a target object with predetermined first feature vectors;
   an ultrasound data acquisition unit operable to transmit ultrasound signals to the target object and receive echo signals reflected from the target object, the ultrasound data acquisition unit being further operable to from ultrasound data based on the receive echo signals;
   an ultrasound image forming unit operable to form an ultrasound image based on the ultrasound data; and
   a processing unit operable to calculate a second feature vector from the ultrasound image and refer to the mapping table to select one of the predetermined first feature vectors closest to the second feature vector, the processing unit being further operable to extract information on the standard view corresponding to the selected predetermined first feature vector from the mapping table.

2.    The ultrasound system of Claim 1, wherein the standard views include a parasternal view, an apical view, a subcostal view and a suprasternal view.

3.    The ultrasound system of Claim 1, wherein the processing unit is operable to calculate the predetermined first feature vectors by:

   establishing first initial vectors from a plurality of typical plane images corresponding to the standard views;
   calculating a mean vector based on the initial vectors;

obtaining first intermediate vectors by subtracting the mean vector from the first initial vectors to thereby form a first intermediate matrix based on the first intermediate vectors;

calculating a first covariance matrix from the first intermediate matrix;

calculating first eigenvalues and eigenvectors by using the first covariance matrix to thereby form a first eigenspace; and

projecting the first intermediate vectors into the first eigenspace to thereby calculating the predetermined first feature vectors.

4. The ultrasound system of Claim 3, wherein the processing unit includes:

an initial vector establishing section operable to establish second initial vectors from the ultrasound image provided from the ultrasound image forming unit;

an intermediate vector forming section operable to subtract the mean vector from the second initial vector to thereby form a second intermediate vector;

a covariance matrix calculating section operable to calculate a second covariance matrix from the second intermediate matrix;

an eigenspace forming section operable to calculate second eigenvalues and

eigenvectors from the second covariance matrix and form a second eigenspace based on the second eigenvectors;

a feature vector calculating section operable to calculate a second feature vector by using the second intermediate vector and the second eigenspace; and

a standard view detecting section operable to refer to the mapping table to select one of the predetermined first feature vectors closest to the second feature vector and extract information on the standard view corresponding to the selected first feature vector from the mapping table.

5. The ultrasound system of Claim 4, wherein the standard view detecting section is operable to compute an Euclidean distance between each of the predetermined first feature vectors and the second feature vector, and select one of the predetermined first feature vector having the smallest Euclidean distance and extract the information on the standard view corresponding to the selected predetermined first feature vector from the mapping table.

6. A method of providing information on one of a plurality of standard views of a target object in an ultrasound system, comprising:

a) storing a mapping table associating information on a plurality of standard views of a target object with predetermined first feature vectors;

b) transmitting ultrasound signals to the target object and receive echo signals reflected from the target object to from ultrasound data based on the receive echo signals;

c) forming an ultrasound image based on the ultrasound data; and

d) calculating a second feature vector from the ultrasound image;

e) referring to the mapping table to select one of the first feature vectors closest to the second feature vector to extract information on the standard view corresponding to the selected predetermined first feature vector from the mapping table.

7. The method of Claim 6, wherein the standard views include a parasternal view, an apical view, a subcostal view and a suprasternal view.

8. The method of Claim 6, wherein the step a) include:

establishing first initial vectors from a plurality of typical plane images corresponding to the standard views;

calculating a mean vector based on the initial vectors;

obtaining first intermediate vectors by subtracting the mean vector from the first initial vectors to thereby form a first intermediate matrix based on the first intermediate vectors;

calculating a first covariance matrix from the first intermediate matrix;

calculating first eigenvalues and eigenvectors by using the first covariance matrix to thereby form a first eigenspace;

calculating the predetermined first feature vectors by projecting the first intermediate vectors into the first eigenspace; and

forming the mapping table associating information on the standard views with the predetermined first feature vectors.

9. The method of Claim 8, wherein the step d) includes:

establishing second initial vectors from the ultrasound image;
subtracting the mean vector from the second initial vector to thereby form a second intermediate vector;
calculating a second covariance matrix from the second intermediate matrix;
calculating second eigenvalues and eigenvectors from the second covariance matrix and form a second eigenspace based on the second eigenvectors; and
calculating the second feature vector by using the second intermediate vector and the second eigenspace.

10. The method of Claim 9, wherein the e) includes:

computing an Euclidean distance between each of the first feature vectors and
the second feature vector;
selecting one of the predetermined first feature vector having the smallest Euclidean distance; and
extracting the information on the standard view corresponding to the selected first feature vector from the mapping table.

## FIG. 1

## FIG. 2

# FIG. 3

START

ESTABLISH INITIAL VECTORS — S310

CALCULATE MEAN VECTOR — S320

CALCULATE INTERMEDIATE VECTORS — S330

FORM MATRIX — S340

CALCULATE COVARIANCE MATRIX — S350

FORM EIGENSPACE — S360

CALCULATE FEATURE VECTORS — S370

FORM MAPPING TABLE — S380

END

EP 2 189 807 A2

# FIG. 4

211

| 225 | 229 | 48 |
|-----|-----|-----|
| 251 | 33 | 238 |
| 0 | 255 | 217 |

212

| 10 | 219 | 24 |
|-----|-----|-----|
| 255 | 18 | 247 |
| 17 | 255 | 2 |

213

| 196 | 35 | 234 |
|-----|-----|-----|
| 232 | 59 | 244 |
| 243 | 57 | 226 |

214

| 255 | 223 | 224 |
|-----|-----|-----|
| 255 | 0 | 255 |
| 249 | 255 | 235 |

# FIG. 5

140

| 141 | 142 | 143 |
|---|---|---|
| INITIAL VECTOR ESTABLISHING SECTION | INTERMEDIATE VECTOR FORMING SECTION | COVARIANCE MATRIX CALCULATING SECTION |

| BASIC VIEW DETECTING SECTION | FEATURE VECTOR CALCULATING SECTION | EIGENSPACE FORMING SECTION |
|---|---|---|
| 146 | 145 | 144 |

# FIG. 6

220

| 20 | 244 | 44 |
|---|---|---|
| 246 | 21 | 244 |
| 4 | 255 | 2 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020080115316